# EUROPEAN PATENT APPLICATION

(11) **EP 3 673 846 A1**
(43) Date of publication of application: **01.07.2020**
(21) Application number: 19180033.3
(22) Date of filing: 13.06.2019
(51) Int. Cl.: A61B 18/14, A61N 1/38, A61B 5/0428, A61B 5/0402, A61M 25/00, A61B 5/042, A61B 5/00, A61N 1/05

(54) **DEVICE FOR AN ELECTROPHYSIOLOGY PROCEDURE**

(30) Priority: 27.12.2018 DE 102018133630
(71) Applicant: Ionescu, Bogdan, 50933 Köln (DE)
(72) Inventor: Ionescu, Bogdan, 50933 Köln (DE)

(57) **Abstract**

An electrophysiological cardiac catheter device comprising a plurality of electrodes which are connected via a selector switch over a resistor to a neutral electrode. Voltage and/or current at one of the electrodes may be measured for cardiac mapping.

## Description

### Field of the invention

The present invention relates to electrophysiology catheters, systems and methods for performing cardiac mapping and/or ablation procedures.

### Description of the related art

EP 1233716 B1 discloses a catheter which has a helical electrode member. The effective length and diameter of this electrode member can be adjusted by actuating a stylet which runs through a hollow, flexible body of the catheter. The stylet runs through hollow, flexible body before exiting the body at aperture. The stylet re-enters the body of the catheter through aperture and in to anchor member.

US 2001/0039413 A1 describes a steerable catheter. The catheter comprises a wire housed within a sheath which is formed of a shape-retentive and resilient material having a curved shape at its distal end. An operator can adjust the shape and radius of curvature of the distal end region.

US 2005/0177053 A1 shows as system for perform an ablation procedure. The handle shown in the system has a safety switch for shutting down the RF generator which delivers current to the heart via the catheter.

### Background

Catheter examination is a special cardiac procedure for patients with cardiac arrhythmias. A catheter exam can determine accurately which form of an arrhythmia is present. Usually the treatment can be performed concurrently to the examination and diagnosis.

The human heart is a very complex organ, who exerts efficient and rhythmical muscle contractions when the electrical impulse flows properly through it. The electrical impulse appears to originate at a specific region inside the right atrium to be further on conducted throughout the heart, first through the atria (from the sinoatrial node down to the atrioventricular node) and then through the ventricles along the specific His-Purkinje system, causing a contraction of the muscles that results in a heartbeat. The atria contract first, followed by the ventricles. This is essential for the proper functioning of the heart.

An irregular propagation of the electrical impulses disrupts the heart's proper function. This disfunction is defined as a cardiac arrhythmia. Arrhythmias can occur if the electrical impulse originating from another regions initiates and maintains abnormal rhythms, what is called an ectopic or focal arrhythmia. Another possibility is, when electrical impulse circulates repetitively in a closed circuit what is called a reentrant arrhythmia.

To prevent these disturbances, medical techniques and equipments have been developed. The techniques and the equipments are used to locate cardiac regions responsible for the cardiac arrhythmias. Afterwards those regions can be disabled rendering arrhythmias unable to reproduce themselves. The common technique known from the state of the art is to apply electrical stimuli from an external stimulator to various portions of the heart tissue to trigger a disorder or an arrhythmia and to detect stepwise the dissipation of the electrical energy throughout the heart. The responsible tissue that provokes or maintains the arrhythmia can thus be identified and subsequently ablated to produce scars that either interrupt the conduction pathways or terminate the focal initiation. In medicine, ablation is the same as removal of a part of biological tissue, usually by surgery.

The regions to be ablated are usually determined by means of a cardiac mapping technique. Mapping typically involves introducing a catheter having one or more electrodes into the patient, in the close proximity of the heart of the patient, by advancing the catheter through a blood vessel and into an inner or endocardial aspect of the heart, or by approaching the outer or epicardial aspect of the heart through a pericardial access. Thus, a continuous, simultaneous recording can be made with a multichannel recorder at each of several different endocardial or epicardial positions. The real time processing of the totality of these recordings from various endocardial and/or epicardial positions allows the creation of a cardiac map on which the regions targeted for the ablation are displayed.

An ablation catheter delivers energy to the tissue for deliberately creating a targeted lesion in the tissue.

One or more suitably positioned lesions will typically create a region of ablated tissue which serves to the elimination of the arrhythmogenic substrate thus disabling the appearance and/or the maintenance of the arrhythmia.

The ablation energy can be, for example, RF, DC, ultrasound, microwave, cold or laser radiation. The RF ablation is carried out by applying radio-frequency (RF) energy to the catheter electrodes.

A RF-ablation catheter with at least one electrode can transmit electric radio-frequency energy to the tissue adjacent to that electrode to create a targeted lesion into the tissue.

The detection of the problematic region and the ablation will be executed with different catheters. For the correct positioning of a treatment catheter (ablation) a special catheter is used. This catheter can very accurately determine the source of pathological signals from the heart. An ablation catheter is capable of examining specific areas.

### Summary of the invention

In view of the prior art, the problem to be solved by the invention is to provide a cardiac electrostimulation device, a simple and robust catheter device for an electrophysiology procedure and a method for an electrophysiology procedure of the heart.

The solution of the problem is described in the independent claims. The dependent claims relate to further improvements of the invention.

A first embodiment relates to a cardiac electrostimulation device and an electrophysiology catheter device adapted for direct tissue contact. The cardiac electrostimulation device may comprise an active electrode device comprising at least one or at least two active electrodes, a neutral electrode, a resistor connected to the neutral electrode and a second selector switch configured to connect the resistor further to at least one of the at least one or at least two active electrodes. It may further comprise a first selector switch configured to connect a sensor device comprising a voltage and/or current sensor to at least one of the at least one or at least two active electrodes. A control unit may be provided and configured to control the first selector switch and the second selector switch dependent on the cardiac signal.

The cardiac electrostimulation device may be an internal compound of a cardiac pacemaker or defibrillator. The electrostimulation device may be configured to connect the resistor to at least one of the at least two active electrodes, if an arrhythmia is detected by the pacemaker or defibrillator. Such therapies can be activated by the device as many times as the arrhythmia presents itself. That involves a repeatedly out-channelling of current out of the heart by the variation of the resistor parameters until the arrhythmia terminates. This configuration may be programmed as part of the conventional programming session of a cardiac pacemaker or defibrillator.

Preferably, the electrophysiology catheter device comprises a catheter, a control unit, a sensor device and a neutral electrode.

When the heart beats too slowly or does not exhibit a spontaneous electric activity at all, the implantable devices like a pacemaker or a defibrillator provide heart stimuli in order to pace the heart to a normal frequency.

When the heart beats too rapidly as during a tachyarrhythmia, pacemakers can react by overdriving that tachyarrhythmia, defibrillators can further on deliver electrical shocks to terminate the tachyarrhythmia if the previous overdriving algorithms proved to be ineffective.

Overdriving a rapid arrhythmia is "overcharging" the heart in an intent to terminate the abnormal propagation.

Switching to a low impedance resistor and outchanneling current from the heart during the arrhythmia is deemed to terminate it too. This treatment has less negative impact and less side effects.

The catheter preferably has a distal end and a proximal end. A flexible shaft connects the distal end and the proximal end. The flexible shaft can be made of a plastic material. The proximal end of the catheter may be designed to be connectable to a handle for operating the functions of the catheter as well as the functions of the catheter device, the control unit or the sensor device.

The distal end of the catheter comprises a plurality of electrodes. The electrodes can be located ring shaped around the distal end with a predefined distance from one electrode to another. Each of the electrodes is connected via a plurality of wires to a plurality of connectors at the proximal end of the catheter. The proximal end of the catheter may have a connecter for connecting the catheter to a handle. The connector may comprise a screw-plug connection for establishing an electrical as well as a mechanical connection.

The control unit which may be contained in a separate housing comprises at least one resistor and at least one signal selector connected to the plurality of connectors at the proximal end of the catheter for setting a current path from the at least one resistor to at least one electrode of the catheter. The signal selector may also be located within the handle. The signal selector may also connect at least one electrode to a sensor device comprising a voltage and/or current sensor, which may be a voltage meter, a current meter or a measuring amplifier. The signal selector may be implemented but not only as semiconductor circuit.

The control handle for operating the functions of the catheter as well as the functions of the catheter device may have at least one operating element to control the resistor and/or the selector. Additionally, the control handle may have an additional operating element to control the catheter respectively the movement of the distal end. The control handle may have at least one electrical connection for connecting the control handle to the catheter and/or the control unit. Preferably, the electrical connection of the catheter corresponds to electrical connection of the control handle. In one embodiment, the control handle can be located in the signal path between the catheter and the control unit.

The sensor device may be part of the control unit and may comprises a voltage and/or a current sensor or -meter. The sensor is connected by the signal selector to at least one of the electrodes which can be the same or another than the electrode connected to the resistor. The configuration of the resistor according to the electrodes and the sensor device is dependent of the measurement task, which has to be executed. In one embodiment, the sensor device is configured to to record the voltage and/or current over time. In other words, the sensor device is able to detect at which time which electrical potential is at a dedicated electrode. In another embodiment, the sensor device can be configured to measure the current channeled out of the heart by one of the electrodes via the resistor. The catheter device may further comprise a control terminal with a display for controlling the measurement results as well as the chosen setting of the catheter device. The screen can be configured as a touch screen to control the functions of the catheter device.

The neutral electrode may be connected to the at least one resistor or the sensor device directly or via at least one selector switch which may be part of the control unit and may be configured to be attached to a patient. The neutral electrode closes the electrical circuit via the at least one electrode at the distal end of the catheter. For attaching the electrode to a patient, the neutral electrode may have an adhesive surface.

The flexible shaft may have several working channels as guide for several tools or electrical, optical or mechanical connections such as wires. The working channels can also be used as media feed-through for example for liquids. The catheter may have a diameter from approximately 1 to 5, preferably from 1 to 3 mm.

The flexible shaft may include at least one wire, which can be formed of a super elastic material and can be shaped to bias the flexible shaft as well as the distal end in several orientations. The super elastic wire may be connected to a control element of the control handle.

As aforementioned, the heart exhibits a spontaneous electrical activity or needs to be stimulated during a regular electrophysiological cardiac procedure by a current produced by an external electrical stimulator in order to deliberately reproduce specific arrhythmias. The invention considers that the heart works as generator for its own in both normal and arrhythmic settings to produce electrical energy that serves for triggering the muscle contraction of the specific muscle regions in the heart. In some settings the abnormal electrical current flow throughout the heart during the arrhythmia may contribute to the perpetuating of the arrhythmia itself rendering it incessant, persistent or permanent or jeopardizing the patient life.

The resistor which can be controlled by the operating element of the control handle enables to channeling current out of the heart via at least one of the electrodes at the distal end. Thus, a lesion respectively an interruption of the current path can be simulated. The amended current dissipation can be recorded by using values from the sensor device respectively the recording over time of the voltage signal curve via the plurality of electrodes. The voltmeter can be attached to the electrodes via the selector or it may have several inputs up to the number of electrodes. Thus, it is possible to measure the voltage between different electrodes as well as the temporal signal dissipation over the electrodes. If a current is channeled out at a specific position of the heart, some electrodes at another position of the distal end will detect an amended or no voltage signal. Current can be channeled out of the heart by connecting a resistor to an electrode via the selector. The value of the resistor can also be varied by on operating element of the handle. In this way the practitioner can determine which position within or at the heart has to be ablated to prevent unwanted current dissipation.

To perform the ablation of the tissue at a specific position with the aforementioned catheter an external generator, which may be an RF surgery generator, can be used to supply energy to the tip assembly of the catheter. The tip assembly then heats up to approximately 60 degrees Celsius. The generator can be connected to the catheter via the electrical connection at the control handle.

The aforementioned catheter will be used for performing an electrophysiology procedure at a human's body.

After a local anesthesia of the groin, a puncture needle is inserted into the vein and at least one catheter is advanced through the inferior vena cava to the heart. The position of the catheter can be checked by fluoroscopy. The access to the heart can also be epicardial. Then the catheter is at the outer surface of the heart.

At various points in the heart, a cardiac electrogram (EGM) may be recorded. During the measurement, the dissipation of electrical signals within the heart will be estimated with the aforementioned catheter.

By varying the value of the variable resistor current can be channeled out of the heart via at least one electrode at the distal end of the catheter to trigger the arrhythmia or to simulate an electrical interruption by channeling current out of the heart. The amended current dissipation can be recorded via the voltmeter respectively the recording overtime of the voltage signal curve via the plurality of electrodes. In this way it is possible to determine the point responsible for the arrhythmia respectively to find points which has to be ablated to prevent an unwanted current dissipation.

If the diagnosis is established, ablation can be performed with an extra ablation catheter if necessary. Ablation can be performed by application of high-frequency current (frequency 300 - 500 kHz, power 10 - 50 watts, with a duration of a single application several seconds to several minutes) to the tip assembly at the working end of the catheter, so that the cardiac tissue responsible for the arrhythmia is destroyed or altered.

To perform the ablation of the tissue, an external generator which may be an RF surgery generator, supplies energy to the tip assembly of the ablation catheter. The tip-tissue assembly then heats up to approximately 60-65 degrees Celsius, resulting in a small scar at this point. Through this scar, the interruption of the undesired current path is achieved. As aforementioned, alternative sources of energy can be employed in order to place the targeted tissue lesion.

When the catheter device is used for an electrophysiologic procedure, identifying the electrically altered region inside the heart during a diagnostic mapping can be instantly followed by electrically addressing that region without deploying of a lesion id.est. without ablation to prevent abnormal dissipation of electricity from that region after the procedure.

Most of the currently used catheters are open irrigated catheters allowing fluid passage from the proximal end to the distal end through their internal lumina and especially directed to the distal tip electrode to prevent heating and clot formation during their use inside the heart and particularly during ablation of the heart tissue.

Instead of purging the catheter with sterile saline, an electrically active dye can be passed through and further on used to first identify the electrically altered region inside the heart which can be consequently "sealed" to assure a permanent current outchanneling from that zone into the circulating blood inside the heart, which may serves as neutral electrode in some settings. This curative "sealing" of the arrhythmia or of the arrhythmic substrate is not ablation and deserves so far an individual name.

### Description of Drawings

In the following, the invention will be described by way of example, without limitation of the general inventive concept, on examples of embodiment with reference to the drawings.
Fig. 1 shows a catheter device.
Fig. 2 shows a schematic diagram of the catheter device.
Fig. 3 shows a sectional view of the heart with a catheter.

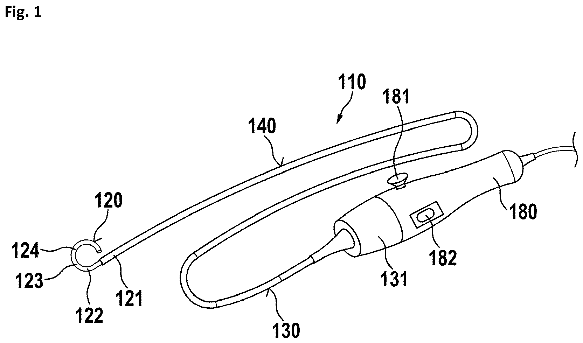

Fig. 1 shows a catheter device 110 with a handle 180. The catheter 110 is divided into three parts. The catheter 110 has a proximal end 130 a flexible shaft 140 and a distal end 120.

The distal end has a plurality of electrodes 121, 122, 123, 124.

The control handle 180 has two operating elements 181, 182 to control the function of the catheter or the catheter device. In this configuration, the control handle 110 can be configured to control the distal end 120 of the catheter 110 via the operating element 181. The control handle 180 can be further configured to trigger different measurement functions of the catheter respectively of the control unit or the sensor device via the operating elements 182. The control handle 180 can have at least one electrical connection for connecting the control handle to the catheter 110 and/or to other parts of the catheter device 100.

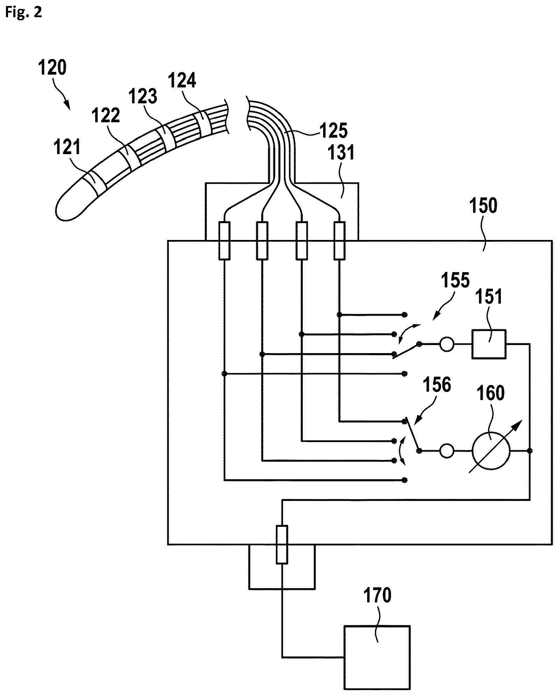

Fig. 2 shows a schematic diagram of the electrophysiology catheter device 100. The catheter 110 has at its distal end 120 a plurality of electrodes 121, 122, 123 and 124. Although here four electrodes are shown, the catheter may have any number of electrodes between 2 and 20, preferably between 6 and 12. These electrodes are connected via wires 125 in the catheter and a connector 131 to a control unit 150. The control unit comprises a first selector switch 155 connected to the electrodes and a resistor 155. It is not necessary that the selector switch is connected to all electrodes. Two electrodes or more are sufficient. The resistor may be a fixed or a variable resistor. It preferably is in the range from 10 to 2000 Ohm, preferably between 200 and 400 Ohm. The control unit comprises a second selector switch 156 connected to the electrodes and a sensor device 160. The sensor device may be a voltage or current meter. The term voltage or current meter includes all devices which are able to measure voltages and/or currents. This includes amplifiers and analog/digital converters which may provide digital information to a computer. It is not necessary that the selector switch is connected to all electrodes. Two electrodes or more may be sufficient.

A neutral electrode is connected to the resistor and/or sensor device.

This neutral electrode 170 allows current from the heart 200 of the patient to flow back to the body. For attaching the neutral electrode 170 to a patient, the neutral electrode 170 may have an adhesive surface.

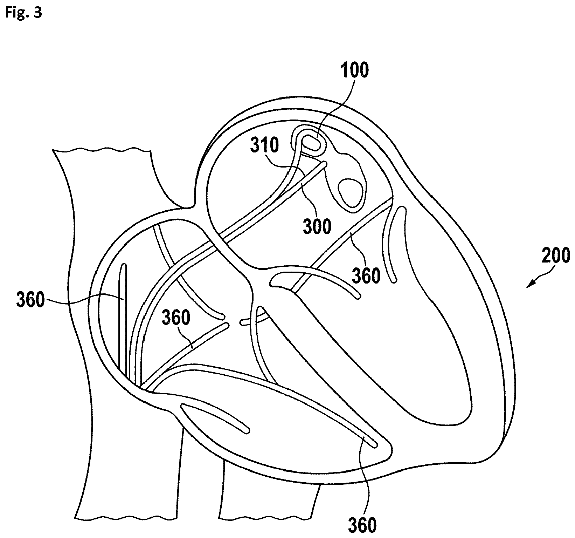

Fig. 3 shows a heart 200 in detail. For the correct positioning of the ablation catheter 300, the inventive catheter 100 can be used. This catheter can very accurately determine the source of morbid signals from the heart 200. By means of an ablation catheter 300, these areas can then be targeted. With the tip assembly 310 of the ablation catheter 300 lesions (scars) in the heart 200 can be set which suppressed the transmission of pathological impulses. An additional catheter 360 can be used to apply an electrical signal to different positions within the heart 200 or to record a cardiac electrogram (EGM).

Although the invention has been illustrated and described in detail by the embodiments explained above, it is not limited to these embodiments. Other variations may be derived by the skilled person without leaving the scope of the attached claims.

In addition, numerical values may include the exact value as well as a usual tolerance interval, unless this is explicitly excluded.

Features shown in the embodiments, in particular in different embodiments, may be combined or substituted without leaving the scope of the invention.

### List of reference numerals

- 100: electrophysiology catheter device
- 110: catheter
- 120: distal end
- 121-124: electrodes
- 125: wires
- 130: proximal end
- 131: connectors
- 140: flexible shaft
- 141: flexible wire
- 150: control unit
- 151: resistor
- 155: first selector switch
- 156: second selector switch
- 160: sensor device
- 161: voltage meter
- 162: current meter
- 170: neutral electrode
- 180: handle
- 181: operating element
- 182: operating element
- 200: heart
- 300: ablation catheter
- 310: tip assembly
- 360: catheter

## Claims

1. A cardiac electrostimulation device comprising:
an active electrode device comprising at least one active electrode,
a neutral electrode,
a sensor device comprising a voltage and/or current sensor, the sensor device being connected to the neutral electrode, and being configured to provide a cardiac signal,
a resistor having a fixed value in the range of 10Ω to 1MΩ or being adjustable within a part of said range, the resistor being connected to the neutral electrode,
a first selector switch connected to at least one of the at least two active electrodes and further connected to the sensor device being configured to electrically connect the sensor device to the at least one of the at least two active electrodes,
a second selector switch connected to at least one of the at least two active electrodes and further connected to the resistor being configured to electrically connect the resistor to the at least one of the at least two active electrodes,
a control unit being configured to control the first selector switch and the second selector switch dependent on the cardiac signal.

2. The cardiac electrostimulation device according to claim 1,
**characterized in, that**
the device is part of a catheter device wherein the neutral electrode is configured to be attached to a patient's body.

3. The cardiac electrostimulation device according to claim 1 or 2,
**characterized in, that**
the control unit is configured to control the second selector switch, such that the resistor is connected to a predetermined electrode after a specific cardiac signal has been provided, wherein the cardiac signal may indicate a cardiac arrhythmia .

4. The cardiac electrostimulation device according to claim 1,
**characterized in, that**
the device is part of an implantable device which further may be a pacemaker or a defibrillator wherein the neutral electrode is either part of the housing of the implantable device or the neutral electrode is configured to be implanted into a patient's body or the device is part of a catheter.

5. The cardiac electrostimulation device according to claim 1 or 2,
**characterized in, that**
the control unit is configured to control the second selector switch based on an external input signal or based on the setting of a configuration switch.

6. An electrophysiology catheter device (100) comprising a cardiac electrostimulation device according to claim 1,
**characterized in, that**
the electrophysiology catheter device comprises a catheter (110),
the catheter (110) comprises a distal end (120) connected by a flexible shaft (140) to a proximal end (130),
the distal end (120) of the catheter (110) comprises the active electrode device (121), each of the active electrodes (121) is connected via a plurality of wires (125) to at least one connector (131) at the proximal end (130),
the neutral electrode (170) is configured to be attached to a patient (400).

7. An electrophysiology catheter device (100) according to claim 6,
**characterized in, that**
the catheter (110) is configured to electrically connect an RF surgery generator or ice, ultrasound or laser external generator to at least one of the active electrodes.

8. An electrophysiology catheter device (100) according to claim 6,
**characterized in, that**
the catheter (110) comprises to a control handle (180) configured to control the sensor device (160) and/or the control unit (150).

9. An electrophysiology catheter device (100) according to any of the previous claims,
**characterized in, that**
the sensor device (160) is configured to record the voltage and/or current over time.

10. An electrophysiology catheter device (100) according to any of the previous claims,
**characterized in, that**
the sensor device (160) is configured to measure the current flowing out of the heart by at least one of the electrodes (121 - 124).

11. An electrophysiology catheter device (100) according to any of the previous claims,
**characterized in, that**
the signal selector (155) is configured to select one of each of the electrodes (121-124) to be connected to the current meter (162) and/or the voltage meter (161).

12. An electrophysiology catheter device (100) according to any of the previous claims,
**characterized in, that**
the flexible shaft (140) of the catheter (100) comprises at least one flexible wire (141) formed of a super elastic material and shaped to bias the distal end (120) in at least one orientation in response to movement of an operating element (181) of the catheter (110).

13. An electrophysiology catheter device (100) according to any of the previous claims,
**characterized in, that**
the handle (180) has an operating element (181) for triggering different measurement functions of the control unit (150) or the sensor device (160).

14. An electrophysiology catheter device (100) according to any of the previous claims,
**characterized in, that**
the control unit (150) or the sensor device (160) is configured to process a set of measurement data delivered by the current meter (162) and/or the voltage meter (161).

15. An electrophysiology catheter device (100) according to any of the previous claims,
**characterized in, that**
the control unit (150) or the sensor device (160) is configured to display the set of measurement data at a display as previously processed by the control unit (150) or the sensor device (160).
